# EUROPÄISCHE PATENTANMELDUNG

(11) **EP 1 319 647 A1**
(43) Veröffentlichungstag der Anmeldung: **18.06.2003**
(21) Anmeldenummer: 02027107.8
(22) Anmeldetag: 04.12.2002
(51) Int. Cl.: C07C 43/11, C08G 65/26, C11D 1/72, C11D 1/74

(54) **Verfahren zur Herstellung alkoxylierter nichtionischer Tenside**

(30) Priorität: 13.12.2001 DE 10161351
(71) Anmelder: Cognis Deutschland GmbH & Co. KG, 40589 Düsseldorf (DE)
(72) Erfinder: Behler, Ansgar, Dr., 46240 Bottrop (DE); Clasen, Frank, 40721 Hilden (DE); Hübner, Norbert, Dr., 40764 Langenfeld (DE); Westfechtel, Alfred, Dr., 40724 Hilden (DE)

(57) **Zusammenfassung**

Die Erfindung betrifft ein Verfahren zur Herstellung alkoxylierter nichtionischer Tenside, bei dem man Verbindungen mit aktiven Wasserstoffatomen oder Carbonsäureester mit Alkylenoxiden in Gegenwart von Metallboranaten als Katalysator sowie ggf. weiteren ausgewählten Co-Katalysatoren umsetzt.

## Beschreibung

Die Erfindung betrifft ein Verfahren zur Herstellung alkoxylierter nichtionischer Tenside, bei dem man Verbindungen mit aktiven Wasserstoffatomen oder Carbonsäureester mit Alkylenoxiden in Gegenwart von Metallboranaten als Katalysator sowie ggf. weiteren ausgewählten Co-Katalysatoren umsetzt.

Eine wichtige Gruppe der nichtionischen Tenside stellen Anlagerungsprodukte von Alkylenoxiden, insbesondere Ethylenoxid und/oder Propylenoxid an Verbindungen mit aktivem Wasserstoff dar, die üblicherweise mittels homogener Katalyse in Gegenwart von Alkalimetallhydroxiden oder Alkalimetallalkoholaten hergestellt werden. Aus derart katalysierten Verfahren werden Produkte mit einer breiten Homologenverteilung erhalten. Produkte mit eingeschränkter Homologenverteilung können erhalten werden, wenn die Umsetzung in Gegenwart von ggf. modifizierten Hydrotalciten, beispielsweise gemäß der deutschen Offenlegungsschrift DE 38 330 76 A erfolgt.

Problematisch wirkt sich bei den aus dem Stand der Technik bekannten Verfahren jedoch aus, daß die erhaltenen Produkte in der Regel eine relativ starke dunkle Verfärbung zeigen. Insbesondere bei der Alkoxylierung ungesättigter Produkte wird eine hohe Zahl an Verfärbend wirkenden Nebenprodukten erhalten. Dies stößt jedoch bei den Anwendern derartiger Produkte, beispielsweise bei den Herstellern von Wasch- oder Reinigungsmitteln, auf Vorbehalte, da die Endverbraucher in der Regel Wert auf ein hellfarbiges, vorzugsweise weißes Produkt legen. Um farblose Tenside zu erhalten, welche die Anforderungen im Hinblick auf die Farbzahl erfüllen, mußten die Alkoxylierungsprodukte in der Regel einem separaten Bleichvorgang unterworfen werden. Ein derartiger zusätzlicher Bleichvorgang ist jedoch sowohl in ökonomischer als auch in ökologischen Hinsicht nachteilig.

Die Aufgabe der vorliegenden Erfindung hat somit darin bestanden, ein Verfahren zur Herstellung von alkoxylierten nichtionischen Tensiden zur Verfügung zu stellen, welches die Nachteile des Standes der Technik nicht mehr aufweist. Insbesondere bestand die Aufgabe der vorliegenden Erfindung darin, ein Verfahren zur Herstellung von alkoxylierten nichtionischen Tensiden zur Verfügung zu stellen, bei dem ohne zusätzliche Verfahrensschritte hellfarbige Produkte erhalten werden. Eine weitere Aufgabe der vorliegenden Erfindung bestand darin, ein Verfahren zur Herstellung von alkoxylierten nichtionischen Tenside zur Verfügung zustellen, bei dem sich auch aus Ausgangsverbindungen mit einer oder mehreren olefinisch ungesättigten Doppelbindungen hellfarbige Verfahrensprodukte erhalten lassen.

Die der vorliegenden Erfindung zugrundeliegende Aufgabe konnte dadurch gelöst werden, daß man die Alkoxylierung in Gegenwart von Metallboranaten durchführt.

Ein Gegenstand der vorliegenden Erfindung ist daher ein Verfahren zur Herstellung alkoxylierter nichtionischer Tenside durch Umsetzung von Verbindungen mit aktiven Wasserstoffatomen oder von Carbonsäureestern mit Alkylenoxiden in Gegenwart mindestens eines Metallboranats.

Als Verbindungen mit aktiven Wasserstoffatomen kommen beispielsweise die folgenden Stoffklassen in Betracht:
a1) Alkohole mit 6 bis 22 Kohlenstoffatomen (sogenannte Fettalkohole), wie z.B. Capronalkohol, Caprylalkohol, Caprinalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Ricinolylalkohol, Elaeostearylalkohol, Arachylalkohol, Gadoleylalkohol, Behenylalkohol, Erucylalkohol sowie deren technische Gemische, wie sie beispielsweise bei der Hydrierung von Methylesterfraktionen nativer Herkunft oder von Aldehyden aus der Roelen'schen Oxosynthese anfallen.
   Als weitere Gruppe von Fettalkoholen kommen ferner auch die sogenannten Guerbetalkohole in Betracht, die durch alkalisch katalysierte Kondensation von 2 Mol Fettalkohol hergestellt werden und 12 bis 36 Kohlenstoffatome enthalten können.
   Bevorzugt werden im Rahmen des erfindungsgemäßen Verfahrens Fettalkohole eingesetzt, die mindestens eine olefinisch ungesättigte Doppelbindung aufweisen.
a2) Carbonsäuren mit 6 bis 22 Kohlenstoffatomen (sogenannte Fettsäuren) und Hydroxyfettsäuren, wie z.B. Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Ricinolsäure, Elaeostearinsäure, Arachinsäure, Gadoleinsäure, Behensäure und Erucasäure sowie deren technische Gemische, wie sie beispielsweise bei der Druckspaltung von natürlichen Fetten und Ölen anfallen. Bevorzugt sind Fettsäuren, die mindestens eine olefinisch ungesättigte Doppelbindung aufweisen.
a3) Alkylphenole, Polyglycole, Fettamine, vicinale hydroxy/alkoxysubstituierte Alkane, die man beispielsweise durch Ringöffnung von Epoxidverbindungen mit Alkoholen oder Carbonsäuren erhalten kann, sowie sekundäre Alkohole. Bevorzugt sind im Rahmen des erfindungsgemäßen Verfahrens Alkylphenole, Polyglycole, Fettamine, vicinale hydroxy/alkoxysubstituierte Alkane, die man beispielsweise durch Ringöffnung von Epoxidverbindungen mit Alkoholen oder Carbonsäuren erhalten kann, sowie sekundäre Alkohole, die mindestens eine olefinisch ungesättigte Doppelbindung aufweisen.

Innerhalb der Gruppe der Verbindungen mit aktiven Wasserstoffatomen werden die olefinisch ungesättigten Alkohole oder Carbonsäuren mit 6 bis 22 Kohlenstoffatomen bevorzugt.

In einer weiteren Ausführungsform des erfindungsgemäßen Verfahrens werden als Ausgangsstoffe Carbonsäureester eingesetzt. Auch hier können grundsätzlich zwei Typen unterschieden werden:
b1) Carbonsäureniedrigalkylester der Formel (I),

   R¹CO-OR² (I)

   in der R¹CO für einen aliphatischen Acylrest mit 6 bis 22 Kohlenstoffatomen und R² für einen linearen oder verzweigten Alkylrest mit 1 bis 4 Kohlenstoffatomen steht.
   Typische Beispiele sind Ester der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Ricinolsäure, Elaeostearinsäure, Arachinsäure, Gadoleinsäure, Behensäure und Erucasäure sowie deren technische Gemische mit Methanol, Ethanol, Propanol oder Butanol. Vorzugsweise werden Methylester von Fettsäuren mit 12 bis 18 Kohlenstoffatomen und insbesondere olefinisch ungesättigte Methylester von Fettsäuren mit 12 bis 18 Kohlenstoffatomen eingesetzt.
   b2) Carbonsäureglycerinester der Formel (II), 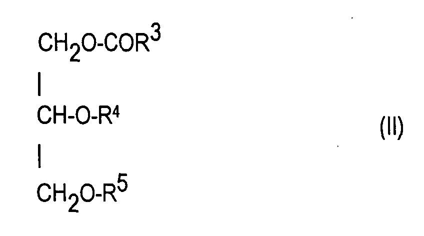 in der R³CO für einen aliphatischen Acylrest mit 6 bis 22 Kohlenstoffatomen und R⁴ und R⁵ unabhängig voneinander für Wasserstoff oder ebenfalls einen aliphatischen Acylrest mit 6 bis 22 Kohlenstoffatomen stehen.
   Typische Beispiele hierfür sind synthetische, vorzugsweise jedoch natürliche Triglyceride, wie Palmöl, Palmkernöl, Kokosöl, Rapsöl, Olivenöl, Sonnenblumenöl, Baumwollsaatöl, Erdnußöl, Leinöl, Lardöl, Rindertalg und Schweineschmalz. Vorzugsweise wird Rizinusöl eingesetzt.

In einer weiteren Ausführungsform der vorliegenden Erfindung werden anstelle der Vollester Fettsäurepartialglyceride, insbesondere Monoglyceride von Fettsäuren mit 12 bis 18 Kohlenstoffatomen eingesetzt. Besonders bevorzugt sind hier technische Kokosfettsäuremonoglyceride oder olefinisch ungesättigte Fettsäurepartialglyceride.

Innerhalb der Gruppe der Carbonsäureester werden Carbonsäureniedrigalkylester, insbesondere die Methylester der Carbonsäuren mit 6 bis 22 Kohlenstoffatomen bevorzugt. Der Begriff "Niedrigalkylester" steht dabei im Rahmen des vorliegenden Textes für lineare oder verzweigte Alkylketten mit 1 bis 8, insbesondere 1 bis 6 oder 1 bis 4 C-Atomen.

Nach dem erfindungsgemäßen Verfahren werden als Alkoxylierungskatalysatoren Alkalimetallborhydride alleine oder in Mischung mit ausgewählten Co-Katalysatoren eingesetzt. Einer Ausführungsform der vorliegenden Erfindung entsprechend wird ausschließlich ein Metallboranat oder ein Gemisch aus zwei oder mehr Metallboranaten als Katalysator eingesetzt.

Als Metallboranate kommen grundsätzlich alle Salze der Mono- oder Polyborane in Frage. Vorzugsweise werden im Rahmen der vorliegenden Erfindung jedoch die Monoboranate eingesetzt. Monoboranate lassen sich in einer dem Fachmann bekannten Weise auf verschiedenen Wegen erhalten (siehe beispielsweise **Hollemann-Wiberg, Lehrbuch der anorganischen Chemie, 91. - 100. Auflage, 1985, S. 834).** Geeignet sind im Rahmen der vorliegenden Erfindung alle Monoboranate, die eine Anlagerung oder Insertion von Alkylenoxiden katalysieren. Insbesondere sind im Rahmen der vorliegenden Erfindung die Alkalimetallborhydride geeignet.

Als Alkalimetallborhydride eignen sich insbesondere die Borhydride von Li, Na oder K, insbesondere LiBH₄ und NaBH₄.

Einer weiteren Ausführungsform der vorliegenden Erfindung entsprechend wird als Alkoxylierungskatalysator Natriumborhydrid eingesetzt.

Als Co-Katalysatoren eignen sich Verbindungen aus der Gruppe, die gebildet wird von Hydroxiden, Oxiden und/oder Alkoxiden von Alkali- und/oder Erdalkalimetallen sowie von Alkali- und/oder Erdalkalisalzen, Zinnsalzen und von Mischmetalloxiden.

Als Hydroxide von Alkali- und/oder Erdalkalihydroxiden eignen sich insbesondere Lithiumhydroxid, Natriumhydroxid, Kaliumhydroxid und/oder Magnesiumhydroxid.

Innerhalb der Gruppe der Oxide von Alkali- und/oder Erdalkalioxiden werden die Oxide des Magnesiums bevorzugt.

Bevorzugte Alkoxide der Alkali- und/oder Erdalkalimetalle sind solche, die sich von kurzkettigen Alkoholen, beispielsweise von solchen mit 1 bis 8 Kohlenstoffatomen und insbesondere von Methanol, Ethanol und/oder 2-Ethylhexanol ableiten. Hierbei sind die Magnesium- und/oder Bariumverbindungen besonders bevorzugt.

Innerhalb der Gruppe der Alkali- und/oder Erdalkalisalze sind die Magnesium- und Bariumsalze, beispielsweise die Carbonate wie Magnesiumcarbonat, oder die Acetate, beispielsweise Magnesiumacetat von besonderer Bedeutung.

Als Mischmetalloxide werden solche Oxidverbindungen bezeichnet, die mindestens zwei verschiedene Metalle enthalten. Bevorzugt ist eines der Metalle Magnesium. Das andere Metall kann Aluminium, Gallium, Zirkon, Indium, Thallium, Kobalt, Scandium, Lanthan und/oder Mangan sein. Besonders bevorzugt werden Magnesium/Aluminiummischoxide. Die Mischoxide können oberflächlich modifiziert sein mit einem oder mehreren der bereits genannten Co-Katalysatoren, insbesondere mit den Hydroxiden und/oder Alkoxiden der Alkali- und/oder Erdalkalimetalle. Derartige Mischmetalloxide und deren Modifizierungsmöglichkeiten werden beispielsweise in der deutschen Offenlegungsschrift DE 44 46 064 A beschrieben.

Sofern die Metallboranate alleinige Katalysatoren sind, werden sie üblicherweise in Mengen von 0,05 bis 3, vorzugsweise in Mengen von 0,1 bis 1 Gew.-% - bezogen auf Ausgangsverbindungen (Verbindungen mit aktivem Wasserstoff bzw. Carbonsäureester und Alkylenoxid) eingesetzt.

Sofern die Metallboranate zusammen mit den ausgewählten Co-Katalysatoren eingesetzt werden, werden sie üblicherweise in Mengen von 0,03 bis 2,5 und insbesondere von 0,1 bis 0,5 Gew.-% - bezogen auf die Ausgangsverbindungen - eingesetzt. Die Co-Katalysatoren können in Mengen von 0,05 bis 5, vorzugsweise in Mengen von 0,1 bis 0,5 und insbesondere in Mengen von 0,1 bis 0,3 Gew.-% - bezogen auf die Ausgangsverbindungen - eingesetzt werden. Die Menge an eingesetzten Co-Katalysatoren sollte im Rahmen der vorliegenden Erfindung jedoch so bemessen werden, daß die Farbzahl der entstehenden Produkte weniger als etwa 100, insbesondere weniger als etwa 50 beträgt. Der Begriff "Farbzahl" bezieht sich im Rahmen des vorliegenden Textes, sofern nicht ausdrücklich etwas anderes erwähnt wird, auf die Hazen-Farbzahl.

Das Verhältnis zwischen Metallboranaten als Katalysator und den gegebenenfalls eingesetzten Co-Katalysatoren kann in weiten Bereichen schwanken, beispielsweise beträgt das Gewichtsverhältnis etwa 5:1 bis etwa 1:5, insbesondere etwa 3:1 bis etwa 1:3 und insbesondere etwa 2:1 bis etwa 1:2.

Die Umsetzung der Verbindungen mit aktiven Wasserstoffatomen bzw. der Carbonsäureester mit den Alkylenoxiden kann in an sich bekannter Weise bei Temperaturen von 120 bis 200 °C, vorzugsweise 150 bis 180 °C und Drücken von 1 bis 5 bar durchgeführt werden. Die Menge des anzulagernden Alkylenoxids ist dabei unkritisch und kann beispielsweise 1 bis 100, vorzugsweise 2 bis 50 und insbesondere 2 bis 20 Mol Alkylenoxid pro Mol H-aktiver Verbindung bzw. Carbonsäureester betragen.

Als Alkylenoxide können Ethylen-, Propylen- und/oder Butylenoxid oder höher Alkylenoxide mit bis zu etwa 22 C-Atomen eingesetzt werden. Vorzugsweise wird im Rahmen des erfindungsgemäßen Verfahrens Ethylenoxid oder Propylenoxid oder deren Gemisch eingesetzt.

Die nach den erfindungsgemäßen Verfahren erhaltenen alkoxylierten nichtionischen Tenside eignen sich als nichtionische Tenside zur Herstellung von Wasch-, Spül und Reinigungsmitteln und zur Herstellung von reinigender Kosmetika, insbesondere von flüssigen Produkten wie flüssigen Textilwaschmitteln, Haarshampoos und dergleichen.

### Beispiele

### Beispiel 1 (erfindungsgemäß)

524 g (entsprechend 2 Mol) eines ungesättigten C16/18-Fettalkohols mit einer Jodzahl von 50 - 55 wurden zusammen mit 3,5 g (entsprechend 0,67 Gew.-% - bezogen auf Ausgangsverbindungen) NaBH₄ in einem Druckbehälter vorgelegt. Der Behälter wurde 30 Minuten bei 120 °C evakuiert und anschließend mit Stickstoff belüftet. Bei max. 180 °C und max. 5 bar Druck erfolgte portionsweise die Zudosierung von 640,4 g (entsprechend 14,55 Mol) Ethylenoxid. Die Reaktionszeit betrug 35 Minuten. Nach Beendigung der Alkoxylierung wurde 60 bei 180 °C nachreagiert und nochmals 30 Minuten bei 120 °C die Apparatur evakuiert.

Es wurde ein ethoxyliertes Produkt mit einer Hazen-Farbzahl von 21 erhalten.

### Beispiel 2 (nicht erfindungsgemäß)

360 g (entsprechend 1,37 Mol) eines ungesättigten C16/18-Fettalkohols mit einer Jodzahl von 50 - 55 wurden zusammen mit 6 g einer 50 Gew.-%igen Lösung (entsprechend 0,83 Gew.-% - bezogen auf Ausgangsverbindungen) KOH in einem Druckbehälter vorgelegt. Der Behälter wurde 30 Minuten bei 120 °C evakuiert und anschließend mit Stickstoff belüftet. Bei max. 180 °C und max. 5 bar Druck erfolgte portionsweise die Zudosierung von 840 g (entsprechend 19,1 Mol) Ethylenoxid. Die Reaktionszeit betrug 28 Minuten. Nach Beendigung der Alkoxylierung wurde 60 Minuten bei 180 °C nachreagiert und nochmals 30 Minuten bei 120 °C die Apparatur evakuiert.

Es wurde ein ethoxyliertes Produkt mit einer Hazen-Farbzahl von 516 erhalten.

## Patentansprüche

1. Verfahren zur Herstellung alkoxylierter nichtionischer Tenside durch Umsetzung von Verbindungen mit aktiven Wasserstoffatomen oder von Carbonsäureestern mit Alkylenoxiden in Gegenwart mindestens eines Metallboranats.

2. Verfahren nach Anspruch 1, **dadurch gekennzeichnet, daß** es in Gegenwart mindestens eines Alkalimetallboranats durchgeführt wird.

3. Verfahren nach Anspruch 1 oder 2, **dadurch gekennzeichnet, daß** die umgesetzten Verbindungen mit aktiven Wasserstoffatomen oder die Carbonsäureester mindestens eine olefinisch ungesättigte Doppelbindung aufweisen.

4. Verfahren nach einem der Ansprüche 1 bis 3, **dadurch gekennzeichnet, daß** die entstehenden Produkte eine Hazen-Farbzahl von weniger als 100 aufweisen.

5. Verfahren nach einem der Ansprüche 1 bis 4, **dadurch gekennzeichnet, daß** die Umsetzung in Gegenwart von NaBH₄ durchgeführt wird.

6. Verfahren nach einem der Ansprüche 1 bis 5, **dadurch gekennzeichnet, daß** als Verbindungen mit aktivem Wasserstoff Alkohole oder Carbonsäuren mit 6 bis 22 Kohlenstoffatomen eingesetzt werden.

7. Verfahren nach einem der Ansprüche 1 bis 5, **dadurch gekennzeichnet, daß** als Carbonsäureester Methylester von Carbonsäuren mit 6 bis 22 Kohlenstoffatomen eingesetzt werden.

8. Verfahren nach einem der Ansprüche 1 bis 7, **dadurch gekennzeichnet, daß** Co-Katalysatoren eingesetzt werden, die ausgewählt sind aus der Gruppe, die gebildet wird von Hydroxiden, Oxiden und/oder Alkoxiden von Alkali- und/oder Erdalkalimetallen sowie von Alkali- und/oder Erdalkalisalzen, Zinnsalzen und von Mischmetalloxiden.

9. Verwendung eines Alkalimetallborhydrids als Katalysator bei der Herstellung alkoxylierter nichtionischer Tenside.
